# EUROPÄISCHE PATENTSCHRIFT

(11) **EP 4 066 765 B1**
(45) Veröffentlichungstag und Bekanntmachung des Hinweises auf die Patenterteilung: **03.09.2025**
(21) Anmeldenummer: 21166320.8
(22) Anmeldetag: 31.03.2021
(51) Int. Cl.: A61B 18/12, A61B 18/00

(54) **AKTIVES ELEKTROCHIRURGISCHES INSTRUMENT**
ACTIVE ELECTROSURGICAL INSTRUMENT
INSTRUMENT ÉLECTRO-CHIRURGICAL ACTIF

(43) Veröffentlichungstag der Anmeldung: 05.10.2022
(73) Patentinhaber: Erbe Elektromedizin GmbH, 72072 Tübingen (DE)
(72) Erfinder: SELIG, Peter, 72147 Nehren (DE); DORNHOF, Konstantin, 78194 Immendingen (DE)
(74) Vertreter: Rüger Abel Patentanwälte PartGmbB

(56) Entgegenhaltungen:
- DE-A1- 2 023 140
- DE-B1- 2 910 196
- US-A- 4 038 984

## Beschreibung

Die Erfindung betrifft ein elektrochirurgisches Instrument mit bestromter Elektrode zur Durchführung elektrochirurgischer Eingriffe an einem menschlichen oder tierischen Patienten.

Elektrochirurgische Instrumente, Sonden oder dergleichen benötigen typischerweise einen elektrochirurgischen Generator zur Speisung des Instruments mit hochfrequentem Wechselstrom. Dazu offenbart die DE 60 2004 009 293 T2 ein elektrochirurgisches System mit einem Generator, an den ein von dem Generator mit Hochfrequenzstrom zu speisendes Instrument anschließbar ist. In einer Ausführungsform weist das Instrument sowohl Koagulationselektroden als auch eine Schneidelektrode auf, die in schneller Folge alternierend beaufschlagt werden, um damit gleichzeitig zu arbeiten. Ein entsprechender elektronischer Umschalter ist dazu in dem Instrument selbst vorgesehen.

Die US 7 896 875 B2 und die US 2011 0112530 beschreiben jeweils ein HF-Instrument, dessen externer Generator von einer Batterie gespeist ist. Die US 9 155 585 B2 beschreibt außerdem einen batteriebetriebenen elektromedizinischen Generator mit fremdgesteuerten Transistoren. Aus der US 2015/0305798 geht ein Instrument mit eingebauter Batterie und eingebautem Generator hervor.

Weiter ist aus der EP 2 572 668 B1 wie auch aus der EP 2 572 669 B1 die Anwendung von Mikrowellen zur medizinischen Behandlung bekannt. Entsprechende Instrumente weisen an einem distalen Ende eines länglichen Schafts eine Mikrowellenantenne auf, die von einem in dem Instrument angeordneten Mikrowellenverstärker gespeist wird. Das Instrument ist über ein Kabel mit einem Mikrowellensignalgenerator verbunden, dessen Signal zu dem Mikrowellenverstärker geleitet wird. Bei einer modifizierten Ausführungsform ist der Mikrowellensignalgenerator in dem Handgriff des Instruments angeordnet. Ein Umschalter ermöglicht dann das Umschalten zwischen Mikrowellensignalen eines externen Signalgenerators und den Mikrowellensignal des internen Signalgenerators.

Auch aus der US 6 039 734 ist ein Instrument mit eingebautem Generator bekannt, das eine monopolare Elektrode zur Behandlung eines Patienten aufweist und den Stromkreis kapazitiv über den Behandler schließt. Die Arbeitsfrequenz ist größer als 5 MHz. Schließlich geht weiterer Stand der Technik aus den US 2017/238987 A1**,** der US 2017/202607 A1**,** der US 2017/079710 A1**,** der US 2016/0270841 A1**,** der US 2014/148803 A1**,** der DE 20 2008 001 365 U1 und der CA 2 286 835 A1 hervor.

Aus der DE 20 23 140 A1 ist eine elektrochirurgisches Gerät zur Speisung eines Instruments bekannt. Das elektrochirurgische Gerät weist einen Gegentaktoszillator zur Erzeugung der für den Betrieb des Instruments nötigen hochfrequenten Spannung auf.

Weiter ist aus der DE 20 10 196 B1 ein Gegentakt-HF-Oszillator für ein Elektrochirurgiegerät bekannt, der mittels einer niedrigen Spannung betreibbar ist.

Während Mikrowellengeneratoren Gewebe durch Einstrahlung von Mikrowellen erwärmen und beeinflussen, arbeiten hochfrequenzbetriebene chirurgische Instrumente mit wesentlich niedrigeren Frequenzen. Die Frequenz des zum Betrieb solcher Instrumente bereitgestellten Stroms liegt typischerweise bei einigen 100 kHz. Solche Instrumente arbeiten mit einem hochfrequenten Stromfluss durch das Gewebe und benötigen dazu immer zwei an dem Gewebe anliegende Elektrode. Die Instrumente dienen der Durchführung verschiedener Maßnahmen, die an den direkten Stromfluss durch das biologische Gewebe geknüpft sind, so zum Beispiel Schneiden, Koagulieren, Fusionieren, Abladieren oder dergleichen. Durch Form und Anwendung entsprechender Elektroden lassen dich die gewünschten chirurgischen Effekte zielgenau beeinflussen. Es kommen dabei verschiedene HF-Spannungen und -Ströme ebenso zur Anwendung wie verschiedene Modulationsformen, beispielsweise unmodulierte HF (CW - "Dauerstrich"), amplitudenmoduliert, beispielsweise gepulst mit oder ohne Pulsbreitenmodulation, und dergleichen mehr. Außerdem können über entsprechende Generatorausgangskennlinien Strom/Spannungs-Zusammenhänge festgelegt werden, die dem Operationserfolg förderlich sind.

Allerdings ist zum Betrieb eines solchen chirurgischen Instruments typischerweise ein externer chirurgischer Generator erforderlich, der die für das Instrument nötigen Modes bereitstellen muss und von dem die Hochfrequenzleistung über ein Kabel zu dem Instrument übertragen wird. Die Modes unterscheiden sich z.B. in Spannung, Strom, Leistung, Modulation und vielem anderem mehr.

Die DE 29 01 153 A1, die US 2010/0137854 A1**,** die US 2011/0245826 A1 und die EP 1 599 146 B1 offenbaren Generatoren mit fremdgesteuerten Schaltern zur Anregung eines oder mehrerer Schwingkreise.

Außerhalb medizinischer Anwendungen, beispielsweise in der Telekommunikationstechnik, werden auch selbstschwingende Generatoren genutzt, wie sie beispielsweise aus der DE 197 80 481 D1**,** der DE 197 80 470 T1**,** der DE 197 19 440 C2 oder der DE 197 19 441 C2 bekannt sind. Diese Generatoren sind als spannungsgesteuerte Gegentaktoszillatoren für den Höchstfrequenzbereich von 1 bis 20 GHz konzipiert. Dabei soll insbesondere mit einer sehr niedrigen Betriebsspannung von beispielsweise lediglich 4,5 V gearbeitet werden. Die Schaltungen eignen sich für den Milliwatt-Bereich. Hingegen wird in der Elektrochirurgie mit erheblich größeren Leistungen und wesentlich höheren Spannungen gearbeitet. Dabei besteht die Gefahr der Spannungsüberlastung einzelner Bauteile.

Es ist Aufgabe der Erfindung, ein verbessertes Instrument bereitzustellen.

Diese Aufgabe wird mit dem Instrument nach Anspruch 1 und ergänzend mit einer Einrichtung nach Anspruch 12 oder 13 gelöst:

Das erfindungsgemäße elektrochirurgische Instrument weist zwei Elektroden zur Einwirkung auf biologisches Gewebe auf. Ist nur eine zum Einwirken auf das Gewebe vorgesehene Elektrode vorhanden, ist mindestens eine zweite Elektrode vorgesehen, die als Neutralelektrode an dem Patienten abseits des Operationsortes anzubringen ist. Das Instrument kann also als monopolares Instrument aufgebaut sein, das eine Elektrode zur Durchführung chirurgischer Eingriffe sowie eine am Patienten zu befestigende Neutralelektrode (oder einen Anschluss für eine Neutralelektrode) aufweist, um den Stromkreis für den Behandlungsstrom zu schließen. An oder in dem Instrument ist ein Hochfrequenzgenerator angeordnet, der als selbstschwingender Oszillator ausgebildet ist. Der Hochfrequenzgenerator bringt die für den chirurgischen Eingriff erforderliche elektrische Leistung auf.

Eingangs- bzw. primärseitig ist der Hochfrequenzgenerator an eine Gleichspannungsquelle oder eine niederfrequente Wechselspannungsquelle (z.B. 50 Hz oder 60 Hz) angeschlossen oder anschließbar. Ausgangs- bzw. sekundärseitig ist der Hochfrequenzgenerator mit den Elektroden verbunden. Die Elektroden können zwei am distalen Ende des Instruments vorgesehene aktive Elektroden oder eine einzelne aktive Elektrode und eine Neutralelektrode sein, die an den Hochfrequenzausgang des Hochfrequenzgenerators angeschlossen sind. Der Hochfrequenzgenerator schwingt mit einer Frequenz zwischen 100 kHz und 10 MHz, typischerweise mehrere 100 kHz zum Beispiel 350 kHz, 500 kHz oder mit einer anderen, im angegebenen Bereich liegenden Frequenz (zum Beispiel 4 oder 5 MHz). Durch die Hochfrequenzerzeugung in dem elektrochirurgischen Instrument entfällt die Notwendigkeit der Weiterleitung hochfrequenter Spannungen und Ströme über längere (mehrere Meter lange) Leitungen, sodass insbesondere auch Abstrahlprobleme und Probleme mit elektromagnetischer Verträglichkeit entfallen.

Es ist möglich, den Hochfrequenzgenerator auf einer einzigen Frequenz schwingen zu lassen, sodass der den Elektroden zufließende Strom ein schmales Spektrum aufweist. Es ist aber auch möglich, den von dem Hochfrequenzgenerator abgegebenen Strom auf eine Weise zu modulieren, dass ein breites Frequenzspektrum erzeugt wird, welches normalerweise bei Fortleitung über elektrische Leitungen wegen des damit verbundenen Antenneneffekts zur Hochfrequenzabstrahlung und somit Störung benachbarter elektrischer Geräte führen kann. Dies ist insbesondere bei einer Puls-Weiten-Modulation mit sehr kurzen Impulsen mit sehr hoher Spannung (mehrere tausend Volt) der Fall. Wegen der unmittelbaren Nähe zwischen dem Hochfrequenzgenerator und den Elektroden können Abstrahlungsprobleme auch bei breitbandigen und leistungsstarken Signalen weitgehend vermieden werden. Es genügt, dem elektrochirurgischen Instrument Gleichspannung oder niederfrequente Wechselspannung zuzuführen, sodass die Zuführungsleitung wenige oder keine elektromagnetische Störstrahlung abstrahlt. Das Kabel zur Stromversorgung des Instruments kann eine ungeschirmte Leitung mit zwei oder mehreren Adern (Leitern) sein. Solche Kabel können wesentlich flexibler sein, als geschirmte Kabel.

Vorzugsweise ist der Hochfrequenzgenerator als Gegentaktoszillator ausgebildet, insbesondere als freischwingender Gegentaktoszillator. "Freischwingend" bedeutet, dass die Schwingung des Gegentaktoszillators in dem Gegentaktoszillator durch positive Rückkopplung aufrechterhalten wird. Es ist möglich, solche Gegentaktoszillatoren mit besonders hohem Wirkungsgrad und sehr geringer Verlustleistung aufzubauen. Dies gilt insbesondere, wenn der Gegentaktoszillator eine Gegentaktkippstufe mit zwei wechselweise schaltenden Transistoren aufweist, an deren Ausgangselektroden erfindungsgemäß jeweils ein Spannungsverstärker angeschlossen ist. Die Ausgangselektroden der Transistoren sind, im Falle der Verwendung von pnp-Transistoren, deren Kollektoren. Werden Feldeffekttransistoren verwendet, handelt es sich bei den Ausgangselektroden um deren Drain-Elektroden. Die nachgeschalteten Spannungsverstärker sind zum Beispiel Bipolartransistoren in Basisschaltung oder Feldeffekttransistoren in Gate-Schaltung. Prinzipiell können als Transistoren, npn-Transistoren, IGBTs, n-oder p-MOSFETs vom Verarmungs- oder Anreicherungstyp, Sperrschicht-FETs, Galliumnitrid-Transistoren (GaN) oder dergleichen Anwendung finden. Durch die Selbststeuerung des Hochfrequenzgenerators schalten die Transistoren der Gegentaktkippstufe nicht überlappend und jeweils im spannungslosen und/oder stromfreien Zustand, sodass die Verlustleistungen an den Transistoren minimal sind. Die angeschlossenen Spannungsverstärker halten die am Hochfrequenzgeneratorausgang anstehenden hohen Spannungen von typischerweise über 100 V von der Kippstufe fern. Die Kippstufe kann mit lediglich 10 oder 20 Volt, also mit niedriger Spannung arbeiten.

Der Hochfrequenzgenerator weist vorzugsweise einen Parallelschwingkreis auf, der aus mindestens einer Spule und mindestens einem Kondensator besteht, die zueinander parallel geschaltet sind. Der Parallelschwingkreis bildet vorzugsweise das frequenzbestimmende Bauelement des Hochfrequenzgenerators, wobei die Rückkopplung zu der Gegentaktkippstufe, über die durch die beiden Spannungsverstärker fließenden Ströme erreicht wird.

Die Auskopplung elektrischer Hochfrequenzenergie aus dem Parallelschwingkreis erfolgt vorzugsweise über eine Auskoppelspule, die in transformatorischer Kopplung zu der Spule des Parallelschwingkreis steht. Der so gebildete Transformator kann für die normgerechte Potentialtrennung zwischen dem Patientenstromkreis und dem Hochfrequenzgenerator ausgelegt sein. Vorzugsweise sind die beiden Enden der Auskoppelspule direkt mit den Elektroden verbunden, die mit dem biologischen Gewebe in Kontakt kommen. Insbesondere sind zwischen der Spule und den Elektroden vorzugsweise keinerlei weitere Bauelemente insbesondere keine Strom- oder Spannungsmesssensoren angeordnet. Dadurch wird die Belastung des Hochfrequenzausgangs des Hochfrequenzgenerators mit Streukapazitäten und die Erzeugung kapazitiver Leckströme minimiert. Durch den Entfall jeglicher HF-seitiger Strom- und Spannungssensorik werden nicht nur kapazitive Leckströme minimiert, sondern es wird auch ein besonders einfacher Schaltungsaufbau erreicht. Es zeigt sich, dass eine Messeinrichtung zur Erfassung von Spannung und/oder Strom, und/oder Leistung und/oder Frequenz gleichspannungsseitig angeordnet werden kann. Entsprechend kann eine entsprechende Messeinrichtung in dem Instrument oder auch in einem speisenden Gerät untergebracht sein. Die Messeinrichtung kann ein Signal erzeugen, das zur Steuerung oder Regelung des Betriebs des Hochfrequenzgenerators dient. Z.B. können die an das biologische Gewebe abgegebene Leistung, der Strom, die Spannung oder andere elektrische Größen reguliert werden. Auch kann anhand des gemessenen Stroms oder anhand der gemessenen Frequenz der Zustand des bestromten Gewebes erfasst und das Instrument entsprechend gesteuert werden. Z.B. kann der Hochfrequenzgenerator abgeschaltet werden, wenn ein Gewebefusionsvorgang beendet ist. Die Beendigung kann anhand des Stroms erfasst werden, wenn dieser eine Schwelle unterschreitet. Alternativ oder ergänzend kann der Hochfrequenzgenerator zeitgesteuert abgeschaltet werden.

Weiter ist es möglich, den Versorgungsspannungseingang des Hochfrequenzgenerators mit einer Spannungsmodulationseinrichtung zu verbinden, die in dem Instrument oder in einem speisenden Gerät untergebracht sein kann. Die Spannungsmodulationseinrichtung kann mit der Messeinrichtung verbunden sein, um HF-Leistung mit einer gewünschten Strom/Spannungs-Charakteristik oder einer gewünschten Modulation bereitzustellen.

Die Gleichspannungsquelle kann somit eine fest vorgegebene Gleichspannung, eine einstellbare zeitlich konstante Gleichspannung oder eine zeitlich oder lastabhängig variierende Gleichspannung abgeben. Weil die Verluste in einem (Gegentakt)Oszillator gering und über den Lastbereich im Wesentlichen konstant sind, bilden die Spannung und die Leistung auf der Primärseite des Hochfrequenzoszillators (Gleichstromseite) die Spannungen, Ströme und Leistungen auf der Hochfrequenzseite (Sekundärseite) ausreichend genau ab. Zur Realisierung einfacher Effekte, wie beispielsweise bipolare Koagulation, kann eine Regelung des Hochfrequenzgenerators, insbesondere seiner Leistung oder seiner Spannung, auch komplett entfallen. Beispielsweise kann dazu der Innenwiderstand des Hochfrequenzgenerators an die entsprechende chirurgische Anwendung angepasst sein. Eine solche Anpassung kann beispielsweise durch Auslegung des Generators oder seiner Komponenten oder dergleichen oder durch Einstellmaßnahmen am Generator erfolgen. Insbesondere kann eine solche Anpassung durch geeignete Festlegung des Windungsverhältnisses der Schwingkreisspule zu der Auskoppelspule erfolgen.

Durch das erfindungsgemäße Konzept wird es möglich, mit Frequenzen von bis zu 5 MHz bei gleichzeitig extrem niedrigem Leistungseintrag durch entsprechende Modulation der HF-Spannung zu arbeiten. Beispielsweise kann der Leistungseintrag durch eine Puls/Pause-Modulation des HF-Signals bei sehr niedrigem Puls/Pause-Verhältnis sehr gering gemacht werden. Ein etwaiges an der Elektrode entstehendes Plasma kann dadurch thermisch kühl gehalten werden, sodass dessen chemischer Effekt medizinisch wirksam ist und der thermische chirurgische Effekt in den Hintergrund tritt oder verschwindet.

Einzelheiten vorteilhafter Ausführungsformen der Erfindung ergeben sich aus Ansprüchen sowie den Figuren der Zeichnung und der zugehörigen Beschreibung. Es zeigen:
Figur 1 eine Einrichtung mit einem Instrument und einem speisenden Gerät, in anschaulicher Darstellung,
Figur 2 die Einrichtung nach Figur 1 in Blockdarstellung,
Figur 3 eine abgewandelte Ausführungsform der Einrichtung nach Figur 1 in Blockdarstellung,
Figur 4 eine weitere abgewandelte Ausführungsform der Einrichtung nach Figur 1, in Blockdarstellung,
Figur 5 das Schaltungsprinzip der Einrichtung nach Figur 1 bis 4 zur Verdeutlichung des Schaltungskonzepts des Hochfrequenzgenerators,
Figur 6 eine detaillierterer Darstellung der Schaltung der Einrichtung nach den Figuren 1 bis 4,
Figur 7 eine weitere abgewandelte Ausführungsform der Einrichtung nach Figur 1, in Blockdarstellung
Figur 8 eine weitere abgewandelte Ausführungsform der Einrichtung nach Figur 1, in Blockdarstellung.

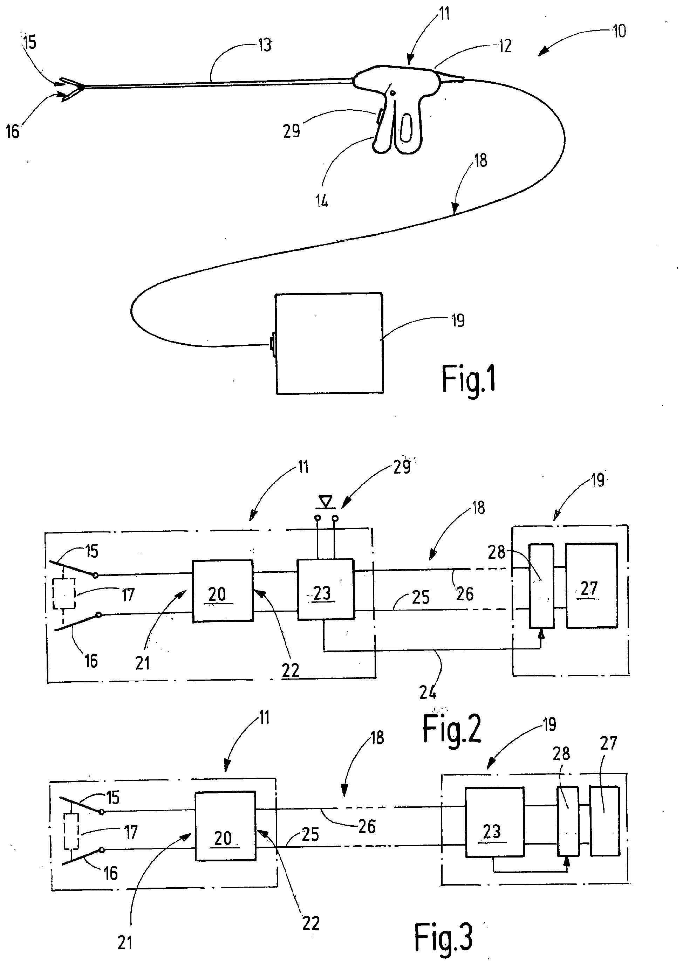

In Figur 1 ist eine Einrichtung 10 zur chirurgischen Einwirkung auf einen Patienten veranschaulicht. Zu der Einrichtung 10 gehört ein Instrument 11, das hier zur Verdeutlichung als laparoskopisches bipolares Instrument veranschaulicht ist. Ausgehend von seinem als Gehäuse ausgebildeten Handgriff 12 erstreckt sich ein Schaft 13, an dessen distalen Ende ein Werkzeug mit zum Beispiel zwei Branchen gehalten ist, die durch Betätigung eines Handhebels 14 nach Art einer Zange geöffnet und geschlossen werden können. An den Branchen sind zum Beispiel an den aufeinander zu weisenden Seiten Elektroden 15, 16 angeordnet, die dazu geeignet sind, zwischen ihnen aufgenommenes und durch Betätigung des Handhebels 14 komprimiertes Gewebe 17 direkt zu bestromen, d.h. einen Strom zwischen den Elektroden 15, 16 durch das Gewebe 17 fließen zu lassen. Das Gewebe 17 ist in den Figuren 2, 3 und 4 jeweils durch einen gestrichelt dargestellten ohmschen Widerstand veranschaulicht.

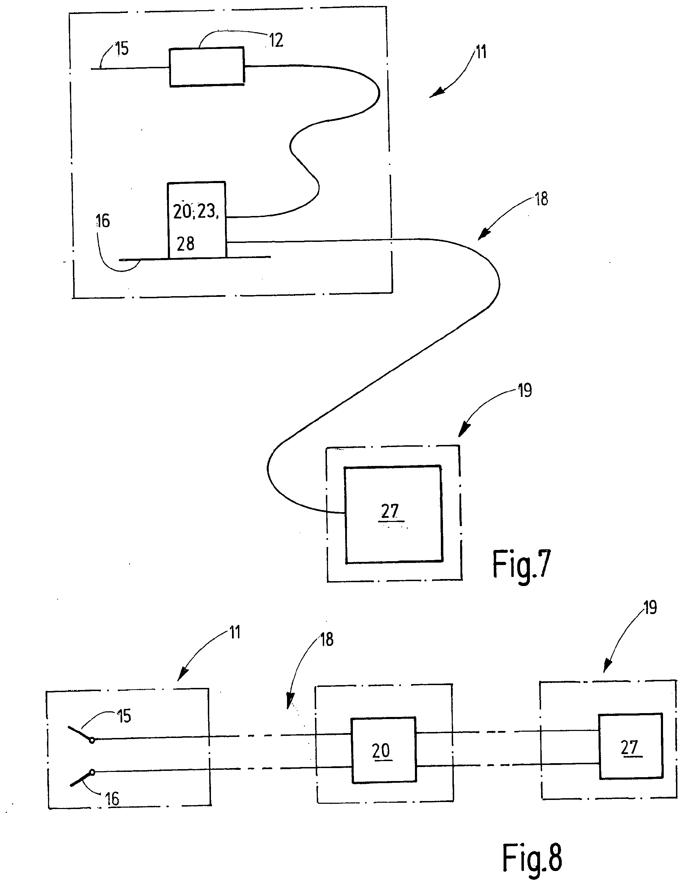

Das Instrument 11 kann prinzipiell auch auf andere nicht dargestellte Weise ausgebildet sein. Insbesondere ist es möglich, es als offenchirurgisches Instrument, beispielsweise als elektrochirurgisches Zangeninstrument auszubilden oder zusätzlich zu den Elektroden 15, 16 ein oder mehrere weitere Elektroden vorzusehen. Beispielsweise können zusätzlich zu den vorzugsweise zur Koagulation des Gewebes 17 eingesetzten Elektroden 15, 16 eine Schneidelektrode oder dergleichen vorgesehen sein. Es ist auch möglich, das Instrument 11 als monopolares Instrument (Figur 7) mit nur einen aktiven Elektrode 15 auszubilden. Dieser aktiven Elektrode 15 ist dann eine Gegenelektrode 16 zugeordnet, die zum Beispiel als großflächige Neutralelektrode ausgebildet ist, die an dem Patienten anzubringen ist, um den Stromkreis zu schließen (Figur 7).

Das Instrument 11 ist über eine Leitung, beispielsweise ein zwei- oder mehradriges vorzugsweise nicht abgeschirmtes Kabel 18 mit einem Gerät 19 verbunden, das zur Speisung des Instruments 11 mit elektrischem Strom dient.

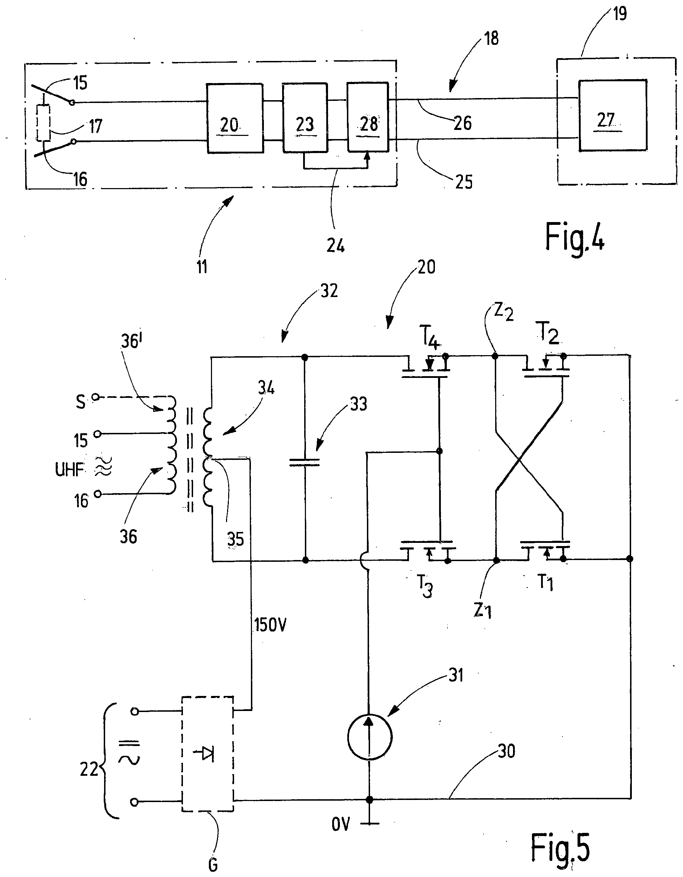

Zur weiteren Erläuterung wird auf Figur 2 verwiesen. Das Instrument 1 und das Gerät 19 sind dort schematisch als strichpunktierte Blöcke dargestellt. Das Instrument 11 enthält einen Hochfrequenzgenerator 20, der einen Hochfrequenzausgang 21 und einen Versorgungsspannungseingang 22 aufweist. Der Hochfrequenzausgang ist an die mindestens zwei Elektroden 15, 16 angeschlossen, um diese mit hochfrequenter Spannung UHF (Figur 5) zu beaufschlagen und einen entsprechend hochfrequenten Strom zu liefern. Die an den Elektroden 15, 16 anliegende Spannung liegt typischerweise in einem Bereich zwischen 100 V und mehreren 100 V. Sie kann in Einzelfällen, zum Beispiel zur Versorgung von Schneidelektroden, auch wesentlich höhere Werte annehmen, z.B. bis zu mehreren 1000 V Spitzenspannung.

Der Versorgungsspannungseingang 22 ist ein Gleichspannungseingang oder, falls ein Gleichrichterblock G vorhanden ist, ein Eingang für niederfrequente Wechselspannung. In diesem Fall kann der Versorgungsspannungseingang 22 je nach verwendeter Gleichrichterschaltung darauf ausgelegt sein, sowohl Gleichspannung als auch niederfrequente Wechselspannung zu akzeptieren. Der Versorgungsspannungseingang 22 ist über entsprechende Leitungen an eine Messeinrichtung 23 angeschlossen, die wenigstens eine physikalische elektrische Größe erfasst, beispielsweise die an dem Versorgungsspannungseingang 22 anliegende Spannung und/oder den dem Versorgungsspannungseingang 22 zufließenden Strom und/oder die dem Versorgungsspannungseingang 22 zufließende Leistung und/oder die Schwingfrequenz des Hochfrequenzgenerators 20. Zur Ermittlung der Schwingfrequenz kann die Messeinrichtung 23 die hochfrequente Welligkeit des dem Hochfrequenzgenerator zufließenden Stroms erfassen und auswerten. Die Frequenz der Welligkeit hängt von der Schwingfrequenz des Hochfrequenzgenerators 20 ab. Die Messeinrichtung 23 kann somit ein oder mehrere der genannten physikalischen Größen erfassen und über eine Signalleitung 24 entsprechende Messwerte an das Gerät 19 liefern. Die Signalleitung 24 kann Teil des Kabels 18 sein, das außerdem noch mindestens zwei Adern 25, 26 zur Stromversorgung des Instruments 11 enthält.

Das Gerät 19 umfasst eine Spannungsquelle 27, die eine Speisespannung von typischerweise 100 V, 150 V, 200 V oder eine andere im Bereich zwischen 12 und 500 V liegende Spannung abgeben kann. Die Spannung kann eine Gleichspannung oder eine entsprechende niederfrequente Wechselspannung sein. Die Spannungsquelle liefert die Spannung mit der erforderlichen Leistung an das Instrument liefern. Die zur Verfügung zu stellende Leistung kann dabei von einigen wenigen Watt bis zum mehreren 100 W gehen und liegt typischerweise im Bereich zwischen 100 W und 300 W. Die elektrische Leistung wird dem Instrument 11 dabei über die Adern 25, 26 des Kabels 18 als Gleichstrom- oder Niederfrequenzleistung zugeführt.

In dem Gerät 19 kann eine Spannungsmodulationseinrichtung 28 vorgesehen sein, die dazu eingerichtet ist, die Höhe der von dem Gerät 19 abgegebenen Speisespannung zu beeinflussen. Die Spannungsmodulationseinrichtung 28 kann Teil der Spannungsquelle 27 sein oder, wie in Figur 2 symbolisch veranschaulicht, als gesonderter Block ausgebildet sein. Die Spannungsquelle 27 kann eine batteriebetriebene Spannungsquelle 27 oder eine netzbetriebene Spannungsquelle 27 sein. Vorzugsweise bewirkt sie eine normgerechte Potentialtrennung zwischen einem Energieversorgungsnetz und der instrumentenseitig bereitgestellten Speisespannung.

Das in Figur 1 und 2 veranschaulichte Instrument 11 und das Gerät 19 arbeiten wie folgt.

Nachdem das Instrument 11 über das Kabel 18 mit dem Gerät 19 verbunden ist, ist das Instrument 11 einsatzbereit. Mittels des Handhebels 14 können die Branchen mit den Elektroden 15, 16 bewegt werden und zwischen einander Gewebe 17 aufnehmen. Es kann nun mittels eines Schalters 29 der Hochfrequenzgenerator 20 aktiviert werden. Beispielsweise kann der Schalter 29 dazu an die Messeinrichtung 23 angeschlossen sein, die daraufhin über die Signalleitung 24 ein Freigabesignal an das Gerät 19 sendet. Das Freigabesignal kann bewirken, dass die Spannungsquelle 27 aktiviert wird und/oder dass die Spannungsmodulationseinrichtung 28 die bereitgestellte Gleich- oder Wechselspannung auf die Adern 25, 26 schaltet und somit Spannung und Strom an das Instrument 11 liefert. Im einfachsten Fall ist die Spannungsmodulationseinrichtung 28 dann lediglich ein Schalter. Anstelle des Schalters 29 kann auch ein Fußschalter oder dergleichen vorgesehen sein, der das Gerät 19 aktiviert oder den Strom zu den Adern 25, 26 freigibt.

Mit Aktivierung der Spannungsquelle 27 erhält der Hochfrequenzgenerator 20 an seinem Versorgungsspannungseingang 22 eine Gleichspannung oder niederfrequente Wechselspannung. Er beginnt hochfrequent zu schwingen und an seinem Hochfrequenzausgang 21 die Behandlungsspannung bzw. den Behandlungsstrom abzugeben. Dieser wird somit in unmittelbarer Nähe zu den Elektroden 15, 16 erzeugt, sodass elektromagnetische Störungen in der Umgebung nicht zu erwarten sind. Dies vermeidet insbesondere die Störung anderer Geräte z.B. bei der Video-Endoskopie und bei Robotik-Anwendungen.

Die Messeinrichtung 23 überwacht zum Beispiel den zu dem Hochfrequenzgenerator 20 gelieferten Strom, der in enger Beziehung zu dem an dem Hochfrequenzausgang 21 abgegebenen Strom steht. Soll beispielsweise ein gewünschter Zusammenhang zwischen Behandlungsdauer und Leistungsabgabe zwischen den Elektroden 15, 16 eingestellt werden, kann die Spannungsmodulationseinrichtung 28 die Stromversorgung des Instruments 11 nach gewünschter Zeit abschalten. Soll beispielsweise ein pulsbreitenmoduliertes HF-Signal an dem Hochfrequenzausgang 21 abgegeben werden, kann die Gleichspannungsmodulationseinrichtung 28 die Höhe der an das Instrument 11 gelieferten Gleich- (oder Wechsel-)spannung modulieren, beispielsweise ein- und ausschalten oder zwischen zwei Werten, zum Beispiel 10 V und 150 V, pendeln lassen. Auch kann die Spannungsmodulationseinrichtung 28 einen gewünschten Innenwiderstand des Hochfrequenzgenerators 20, d.h. eine gewünschte Spannungs/Strom-Kurve, erzeugen, beispielsweise indem die an die Adern 25, 26 abgegebenen Spannung mit zunehmenden Strom nach einer gewünschten Funktion reduziert wird. Ist die Messeinrichtung 23 eine Strommesseinrichtung und liefert die Signalleitung 24 das Strommesssignal an die Spannungsmodulationseinrichtung 28, kann die Spannungsmodulationseinrichtung 28 die Spannung in Abhängigkeit vom gemessenen Strom in gewünschter Weise, beispielsweise nach eine Sollkurve, einstellen. Arbeitet der Hochfrequenzgenerator 20 mit konstantem Wirkungsgrad, wird so an dessen Hochfrequenzausgang 21 die gewünschte Charakteristik, d.h. der gewünschte Zusammenhang zwischen HF-Ausgangsspannung und HF-Ausgangsstrom eingestellt.

Das Kabel 28 führt Gleichspannung und Gleichstrom (oder niederfrequente Wechselspannung und Wechselstrom), der allenfalls mit niedriger Frequenz von zum Beispiel einigen Hertz oder einigen kHz oder einigen 10 kHz moduliert sein kann. Eine Störabstrahlung oder Abfluss kapazitiver Leckströme ist über das Kabel 18 nicht gegeben.

Das vorgestellte Konzept kann in mehrerlei Hinsicht variiert werden. Eine erste Variation zeigt Figur 3. Das Instrument 11 nach Figur 3 umfasst den Hochfrequenzgenerator 20 und die Elektroden 15, 16. Im Übrigen gilt die obige Beschreibung entsprechend mit Maßgabe folgender Abweichungen:

Die Messeinrichtung 23 ist nicht in dem Instrument 11, sondern in dem Gerät 19 untergebracht. Infolgedessen enthält das Kabel 18 zwar die Adern 25, 26, eine Signalleitung ist jedoch nicht nötig. Damit kann das Kabel 18 eine einfache zweiadrige ungeschirmte Leitung sein. Ein Aktivierungsschalter ist in Figur 3 nicht veranschaulicht. Er kann als Fußschalter ausgebildet und direkt mit dem Gerät 19 verbunden sein, um die Abgabe von Gleichspannung freizugeben oder zu sperren. Es ist auch möglich, einen Aktivierungsschalter 29 an dem Instrument 11 anzubringen und mit diesem beispielsweise über eine Signalleitung das Gerät 19 zu steuern. Weiter ist es möglich, einen Aktivierungsschalter 29 leidglich als Ein/Aus-Schalter in eine oder beide Adern 25, 26 zu legen, um die von dem Gerät 19 über das Kabel 18 gelieferte Spannung an den Versorgungsspannungseingang 22 zu legen oder von diesem zu trennen.

Eine weitere Modifikation zeigt Figur 4. In dieser Ausführungsform sind in dem Instrument 11 sowohl der Hochfrequenzgenerator 20 als auch die Messeinrichtung 23 und auch die Gleichspannungsmodulationseinrichtung 28 angeordnet. Das Gerät 19 enthält lediglich die Spannungsquelle 27, die zur Abgabe einer festen Gleichspannung (oder niederfrequenten Wechselspannung) eingerichtet ist. Die Spannungsquelle 27 kann beispielsweise ein gewöhnliches Gleichspannungsnetzteil, zum Beispiel ein Hochleistungs-USB-Netzteil oder eine am Operationstisch vorgesehene Spannungsquelle, beispielsweise eine Steckdose für Gleichspannung sein. Es ist auch möglich, als Spannungsquelle 27 eine Wechselspannungsquelle, beispielsweise mit einer Wechselspannung von 50 Hz oder 60 Hz vorzusehen und der Spannungsmodulationseinrichtung 28 noch den Gleichrichterblock G vorzuschalten. Ansonsten gilt hinsichtlich der Funktion des aus dem Instrument 11 dem Kabel 18 und dem Gerät 19 bestehenden Systems die obige Beschreibung zu den vorigen Ausführungsformen entsprechend.

Die Struktur des Hochfrequenzgenerators 20 ist prinzipiell in Figur 5 erläutert. Der Hochfrequenzgenerator 20 ist als Gegentaktoszillator mit insgesamt mindestens vier Schalttransistoren T1, T2, T3, T4 aufgebaut, die vorzugsweise als Feldeffekttransistoren (und/oder GaN-Transistoren) aufgebaut sind (vorzugsweisen n-Kanal, Anreicherungstyp, d.h. selbstsperrend). Prinzipiell können aber auch andere Transistoren in gleicher Schaltungsanordnung verwendet werden beispielsweise, bei umgekehrter Spannungspolarität, p-Kanal Feldeffekttransistoren oder auch Bipolartransistoren (npn oder pnp) IGBTs oder dergleichen.

Die Transistoren T1 und T2 sind mit ihren Source-Elektroden an ein gemeinsames Bezugspotential 30 (Masse) angeschlossen. Die Drain-Elektroden bilden jeweils einen Zwischenabgriff Z1 und Z2. Die Gates der beiden Transistoren T1 und T2 sind mit dem Zwischenabgriff des jeweils anderen Transistors verbunden. Die Transistoren T1 und T2 bilden somit gemeinsam eine Kippstufe mit zwei im Gegentakt schaltenden Transistoren T1 und T2. An den Zwischenabgriffen Z1 und Z2 liegen Rechteckspannungen zwischen Null und wenigen Volt (z.B. 20 V) an, wobei die Transistoren T1 und T2 nie gleichzeitig an oder gleichzeitig aus sind.

An den Zwischenabgriffen Z1 und Z2 sind die Stromeingänge der als Spannungsverstärker in Gate-Schaltung arbeitenden Transistoren T3 und T4 angeschlossen. Die Stromeingänge werden durch deren Source-Elektroden gebildet. Die beiden Gates der Transistoren T3 und T4 sind mit einer festen Spannung verbunden, die über eine Konstantspannungsschaltung 31 bereitgestellt wird.

Die Drain-Elektroden der beiden Transistoren T3 und T4 bilden den Verstärkerausgang, der mit dem Parallelschwingkreis 32 verbunden ist. Dieser besteht aus einem Kondensator 33 (oder mehreren z.B. in Reihenschaltung angeordneten Kondensatoren) und einer Spule 34 (oder mehreren in Reihe geschalteten Spulen). Die Spule 34 weist einen Mittelabgriff 35 auf, der mit dem positiven Potential des Versorgungsspannungseingangs 22 verbunden ist.

Zum Auskoppeln von HF-Leistung aus dem Parallelschwingkreis 32 dient eine Auskoppelspule 36, die transformatorisch mit der Spule 34 koppelt. Die Spule 36 ist ohne Zwischenschaltung weiterer Bauelemente mit den Elektroden 15, 16 verbunden und gibt somit HF-Leistung an diese ab. Bedarfsweise kann eine weitere Auskoppelspule 36' vorgesehen sein, die zur Speisung weiterer Elektroden beispielsweise einer Schneidelektrode S dient, die nicht weiter veranschaulicht ist. Sie kann zum Beispiel in einer der Branchen des Instruments 11 untergebracht sein. Die Spule 36' kann mit der Spule 36 in Reihe geschaltet sein, um eine erhöhte Spannung abzugeben. Es ist auch möglich, eine andere Spulenkonfiguration zu wählen. Die Auskoppelspule bildet zusammen mit dem zwischen den Elektroden 15, 16 gefassten Gewebe 17 vorzugsweise einen galvanischen Stromkreis ohne Verzweigungen.

Die Transistoren T1 und T3 bilden zusammen eine Kaskodeschaltung. Ebenso bilden die Transistoren T2 und T4 zusammen eine Kaskodeschaltung. Der Parallelschwingkreis 32 bildet zusammen mit den beiden Kaskodeschaltungen einen Gegentaktoszillator mit dem Parallelschwingkreis 32, der die Schwingfrequenz der Gegentaktkippschaltung T1, T2 festlegt. Der Gegentaktoszillator kann strukturell und von der Bemessung her vollkommen symmetrisch oder, wie es bevorzugt wird, auch leicht asymmetrisch aufgebaut sein. Die Asymmetrie kann in Bauteilvariationen, insbesondere hinsichtlich der Transistoren, in einer leichten Spulenasymmetrie (Mittelanzapfung der Schwingkreisspule ist nicht genau mittig), in unterschiedlichen Streukapazitäten oder ähnlichem liegen. Dies kann z.B. das Anschwingen des Hochfrequenzoszillators fördern.

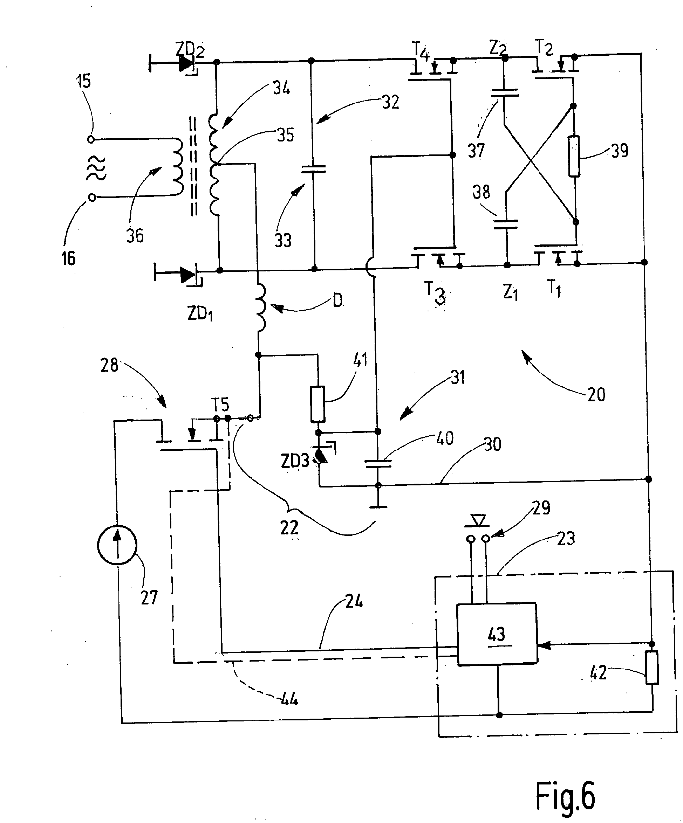

Figur 6 veranschaulicht den Hochfrequenzgenerator 20 nach Figur 5 in etwas detaillierter Darstellung. Auf der obigen Beschreibung der Schaltung nach Figur 5 aufbauend wird ergänzend darauf hingewiesen, dass die aus den Transistoren T1 und T2 gebildete Gegentaktkippschaltung eine kapazitive Kopplung aufweisen kann, indem die Zwischenabgriffe Z1 und Z2 jeweils über Kondensatoren 37, 38 mit den Gates der Transistoren T1 und T2 verbunden sind. Außerdem können die beiden Gates über einen Widerstand 39 untereinander verbunden sein, um im zeitlichen Mittel auf gleichem Potential gehalten zu werden. Vorzugsweise legen die Kondensatoren 37, 38 zusammen mit dem Widerstand 39 eine Kippfrequenz der aus den Transistoren T1 und T2 gebildeten Gegentaktkippstufe fest, die niedriger ist, als die von dem Parallelschwingkreis vorgegebene Schwingfrequenz. Die Transistoren T1 bis T4 können in einem gemeinsamen Gehäuse untergebracht sein und bedürfen normalerweise keiner Kühlung; sie sind ungekühlt.

Der Parallelschwingkreis kann über zwei Z-Dioden ZD1 und ZD2 mit dem Bezugspotential 30 verbunden sein, um Überspannungen an dem Parallelschwingkreis 32 zu verhindern.

Die Konstantspannungsschaltung 31 kann aus einer Parallelschaltung einer Z-Diode ZD3 mit einem Kondensator 40 gebildet sein, denen über einen Widerstand 41 Strom zugeführt wird.

Die Schaltung nach Figur 6 enthält außer dem Hochfrequenzgenerator 20 auch die Messeinrichtung 23, hier beispielhaft durch einen Shunt 42 vertreten. Dieser wird durch einen niederohmigen Widerstand gebildet, der in einer von der Spannungsquelle 27 zu dem Hochfrequenzoszillator 20 führenden Leitung liegt. Zu der Messeinrichtung 23 gehört außerdem ein Block 43, der zur Strommessung die über den Shunt 42 abfallende Spannung abgreift und ein entsprechendes Steuersignal an die Spannungsmodulationseinrichtung 28 liefert. Über eine Leitung 44 kann der Block 43 auch die an dem Spannungseingang 22 anliegende Spannung erfassen.

Die Spannungsmodulationseinrichtung 28 kann durch einen Transistor T5 gebildet sein, dessen Drain-Source-Strecke (oder Kollektor-Emitter-Strecke) in einer von der Spannungsquelle 27 zu dem Versorgungsspannungseingang 22 führenden Leitung liegt. Die Signalleitung 24 kann mit dem Gate des Transistors T5 verbunden sein.

Zur Glättung des von der Spannungsquelle 27 an den Hochfrequenzgenerator 20 gelieferten Stroms kann in der zu der Mittelabzapfung 35 führenden Leitung eine Drossel D vorgesehen sein. Außerdem kann an dem Spannungseingang (hinter dem Gleichrichterblock G, falls vorhanden) ein Pufferkondensator vorgesehen sein.

Der Block 43 kann die gewünschte Funktion der Hochfrequenzgenerators 20 steuern. Solange der Hochfrequenzgenerator 20 über den Transistor T5 eine konstante Spannung erhält, liefert er an seinem Ausgang eine bestimmte Hochfrequenzspannung zur Bestromung der Elektroden 15, 16. Der den Elektroden 15, 16 zufließende Strom wird durch den Shunt 42 und den Block 43 erfasst. Der Block 43 kann einen gewünschten Strom/Spannungs-Zusammenhang festlegen. Es ist möglich vorzusehen, dass verschiedene Strom/Spannungs-Zusammenhänge bereitgestellt und auswählbar sind. Soll beispielsweise bei zunehmendem Strom die Generatorausgangsspannung verringert werden, kann der Block 43 den Transistor T5 über die Leitung 24 entsprechend ansteuern. Der Block 43 kann über eine in Figur 6 gestrichelt dargestellte Leitung mit dem Versorgungsspannungseingang 22 verbunden sein und die dort anliegende Spannung messen. Die gemessene Spannung kann zur Steuerung des Transistors T5 genutzt werden. Der Transistor T5 kann analog oder im gepulsten Betrieb (ein/aus) arbeiten. Durch entsprechendes Freigeben oder Sperren des Transistors T5 über die Leitung 24 kann der Hochfrequenzgenerator 20 ein- und ausgeschaltet oder zwischen hoher Leistung und niedriger Leistung umgeschaltet werden. Andere Modulationsarten sind möglich.

Das erfindungsgemäße Konzept hat eine ganze Reihe von Vorzügen. Der Gegentaktoszillator nach Figur 5 und 6 gestattet eine Realisierung in besonders kleiner Bauform. Es ist keine Kühlung der Transistoren T1 bis T4 erforderlich und zwar auch dann nicht, wenn der Hochfrequenzgenerator Leistung oberhalb von 100 W abgibt. Weiter kann jegliche Strom- und Spannungssensorik im Patientenstromkreis, d.h. auf der Hochfrequenzseite des Hochfrequenzgenerators 20 entfallen. Der Patientenstromkreis ist ein unverzweigter Stromkreis. Die Messeinrichtung 23 (sowie bedarfsweise weitere Messeinrichtungen) können im Gleichstromkreis vorgesehen sein. Selbst die Schwingfrequenz des Hochfrequenzgenerators 20 kann zum Beispiel an dem Shunt 42 infolge der dort zu verzeichnenden Stromwelligkeit erfasst werden. Strom, Spannung und Leistung auf der Primärseite bilden die HF-Spannung auf der Patientenseite ausreichend genau ab. Dies weil die Verluste in dem Hochfrequenzgenerator 20 gering und über seinen Lastbereich im Wesentlichen konstant sind. Somit lässt sich eine Regelung der HF-Spannung des HF-Stroms oder HF-Leistung mit Hilfe der gleichstromseitig gewonnenen primären Messwerte realisieren. Durch den Entfall der HF-seitigen Strom- und Spannungssensorik reduzieren sich auch die Koppelkapazitäten über die sehr große normativ vorgegebene Trennstrecke zwischen dem Gleichstromkreis und dem HF-Stromkreis. Damit reduzieren sich die hochfrequenten Leckströme des Systems.

Zur Realisierung einfachere Effekte, wie beispielsweise einer bipolaren Koagulation, kann eine Regelung auch komplett entfallen. Zum Beispiel kann dazu die Leistungskurve des ungeregelten Hochfrequenzgenerators 20 an die chirurgische Anwendung angepasst sein. Die Lastimpedanz (das ist Widerstand des biologischen Gewebes 17) bestimmt dann den fließenden Strom. Entsprechende Anpassungen können durch Modifikation der Ausgansbeschaltung des Generators, beispielsweise dem Windungsverhältnis der Spulen 34, 36 zueinander oder durch entsprechende Festlegung des Kopplungsfaktors zwischen den Spulen 34, 36 erfolgen. Durch geeignete Bemessung des Kopplungsfaktors zwischen der Schwingkreisspule 34 und der Auskoppelspule 36, durch das L/C-Verhältnis des Schwingkreises, durch geeignete Bemessung der Drossel D kann der Innenwiderstand des Hochfrequenzgenerators, d.h. die Abhängigkeit des HF-Stroms von der durch das Gewebe 17 gebildeten Last festgelegt werden.

Eine solche Anpassung kann auch durch Eingriff an anderer Stelle erfolgen, beispielsweise durch Veränderung der Gate-Vorspannung der Gates der Transistoren T3, T4, erfolgen. Zudem ist keine aufwendige Überwachung von besonderen Lastzuständen, wie Kurzschluss oder Leerlauf, erforderlich. Es ergibt sich ein gegenüber bekannten Generatoren wesentlich vereinfachtes Design. Auch ist keine komplexe Nachführung der Frequenz durch die Ansteuerung notwendig, wie es bei bekannten Generatoren der Fall ist. Das selbstschwingende System, d.h. der Hochfrequenzgenerator 20 benötigt weder externe Taktgeber noch besondere Überwachungsschaltungen.

Der Hochfrequenzgenerator 20 kann auch, wie es in Figur 7 veranschaulicht ist, an der Neutralelektrode 16 angeordnet sein, die somit als Teil des Instruments 11 zu betrachten ist. Die vorige Beschreibung der Ausführungsformen nach Figur 1 bis 6 gilt unter Zugrundelegung der bereits eingeführten Bezugszeichen entsprechend. Bei allen ausführungsformen bietet die Platzierung des Hochfrequenzgenerators 20 in dem Instrument 11 oder nahe bei dem Instrument 11 auch die Möglichkeit, Generatoren mit höheren Frequenzen z.B. 4 MHz sinnvoll zu nutzen. Selbst bei solchen abstrahlungsfreudigen Frequenzen sind die ausgehenden Störungen und kapazitiven Leckströme bei dem erfindungsgemäßen Konzept gering.

Figur 8 veranschaulicht eine zweckmäßige Ausführungsform der Erfindung mit einem Instrument 11, das mit dem Hochfrequenzgenerator 20 lösbar verbunden ist, dieser kann beispielsweise direkt an das Gehäuse des Instruments 11 ansteckbar oder im Verlauf des Kabels 18 angeordnet sein. Beispielsweise kann der Hochfrequenzgenerator 20 auch in dem proximalen Stecker des Kabels 18 angeordnet sein, mit dem das Kabel 18 an das Gerät 19 anzuschließen ist. Es kann sich bei dem Instrument nach Figur 8 um einen ungeregelt arbeitenden Hochfrequenzgenerator 20 handeln, dessen HF-ausgangsseitiger Innenwiderstand an den gewünschten chirurgischen Effekt zum Beispiel Koagulation oder Gewebefusion angepasst ist. Der Innenwiderstand des Hochfrequenzgenerators 20 kann linear oder nicht linear sein. Er kann durch entsprechende Bemessung des Kupplungsfaktors zwischen den Spulen 34, 36 (Figur 5 und 6), durch Bemessung einer entsprechenden Größe der Drossel D (Figur 6) oder auch durch entsprechende Festlegung des Innenwiderstands der Spannungsquelle 18 wie gewünscht festgelegt werden. Bei der Ausführungsform nach Figur 8 können die Messeinrichtung 23 und die Spannungsmodulationseinrichtung 28 entfallen. Es ist aber auch möglich, die Messeinrichtung 23 und die Spanungsmodulationseinrichtung 28 entweder unmittelbar an dem Hochfrequenzgenerator 20 oder alternativ in dem Gerät 19 unterzubringen wie es in Figur 3 zugrunde gelegt ist. Bei allen solchen Abwandlungen, die auf der Ausführungsform nach Figur 8 beruhen, kann der Generator in dem dann von dem Instrument 11 abnehmbaren und somit wieder verwendbaren Instrumentenkabel 18 angebracht sein. Das Instrument 11 kann dann für lediglich einmaligen Gebrauch und nachfolgende Entsorgung vorgesehen sein. Der Generator 29 kann auch in einem separaten Gehäuse als Zwischenstecker oder Zwischenkabel vorgesehen sein, der zwischen dem Instrument 11 und seinem Kabel 18 und dem Gerät 19 anzubringen ist. Auch kann der Hochfrequenzgenerator 20 in einem separaten abnehmbaren Gehäuse an oder in der Nähe der Neutralelektrode 16 vorgesehen sein, wie es Figur 7 anregt.

Das erfindungsgemäße elektrochirurgische Instrument 11 weist mindestens eine Elektrode 15, 16 zur elektrischen Einwirkung auf biologisches Gewebe auf. Die Elektrode ist mit einem Hochfrequenzgenerator 20 gekoppelt, der in unmittelbarer Nähe zu der Elektrode 15 und/oder 16 angeordnet ist. Der Hochfrequenzgenerator schwingt eigengesteuert auf eine Frequenz zwischen 100 kHz und 10 MHz und wird vorzugsweise von einer konstanten oder einer zeitlich schwankenden Gleichspannung gespeist. Das Instrument 11 ist somit über eine Niederfrequenz- oder Gleichspannung führende Leitung mit einer speisenden Quelle beispielsweise einem Gerät 19 verbunden.

### Bezugszeichen:

- 10: Einrichtung
- 11: Instrument
- 12: Handgriff
- 13: Schaft
- 14: Handhebel
- 15: Elektrode
- 16: Elektrode oder Neutralelektrode
- 17: biologisches Gewebe
- 18: Kabel
- 19: Gerät
- 20: Hochfrequenzgenerator
- 21: Hochfrequenzausgang
- 22: Versorgungsspannungseingang
- G: optionaler Gleichrichterblock
- UHF: hochfrequente Spannung
- 23: Messeinrichtung
- 24: Signalleitung
- 25, 26: Adern des Kabels 18
- 27: Spannungsquelle
- 28: Spannungsmodulationseinrichtung
- 29: Schalter
- 30: Bezugspotential
- T1 - T4: Transistoren
- 31: Konstantspannungsschaltung
- 32: Parallelschwingkreis
- 33: Kondensator
- 34: Spule
- 35: Mittelanzapfung
- 36, 36': Auskoppelspule(n)
- S: Schneidelektrode
- 37, 38: Kondensatoren
- 39: Widerstand
- ZD1 - ZD4: Z-Dioden
- 40: Kondensator
- 41: Widerstand
- 42: Shunt
- T5: Transistor
- D: Drossel
- 43: Block, Messeinrichtung
- 44: Leitung

## Patentansprüche

1. Elektrochirurgisches Instrument (11) zur Behandlung menschlicher oder tierischer Patienten,
mit mindestens zwei Elektroden (15, 16) zur Bestromung von biologischem Gewebe (17),
mit mindestens einem als Gegentaktoszillator ausgebildeten Hochfrequenzgenerator (20), der einen Versorgungsspannungseingang (22) und einen Hochfrequenzausgang (21) aufweist, der an die Elektrode (15, 16) angeschlossen ist, wobei der Hochfrequenzgenerator (20) dazu eingerichtet ist, eine zeitlich konstante oder zeitlich schwankende Versorgungsspannung in eine hochfrequente Wechselspannung umzusetzen,
mit einer an eine Spannungsquelle (27) anschließbaren Leitung (18) zur Stromversorgung des Hochfrequenzgenerator (20) an seinem Versorgungsspannungseingang (22),
**dadurch gekennzeichnet, dass** der Gegentaktoszillator eine Gegentaktkippstufe mit zwei wechselweise schaltenden Transistoren (T1, T2) aufweist, an deren Ausgangselektroden jeweils ein Spannungsverstärker angeschlossen ist.

2. Instrument nach Anspruch 1, **dadurch gekennzeichnet, dass** der Hochfrequenzgenerator (20) dazu ausgebildet ist, eine Spannung (UHF) mit einer zwischen 100 kHz und 10 MHz liegenden Frequenz bereitzustellen.

3. Instrument nach einem der vorstehenden Ansprüche,
**dadurch gekennzeichnet, dass** die Spannungsverstärker Transistoren (T3, T4) in Gate- oder Basisschaltung sind.

4. Instrument nach einem der vorstehenden Ansprüche,
**dadurch gekennzeichnet, dass** der Hochfrequenzgenerator (20) einen Parallelschwingkreis (32), bestehend aus mindestens einer Spule (34) und mindestens einem Kondensator (33) aufweist, die zueinander parallel geschaltet sind, und dass der Hochfrequenzgenerator (20) einen Auskoppelkreis aufweist, der ausschließlich an die Elektroden (15, 16) zur elektrischen Einwirkung auf das biologisches Gewebe (17) angeschlossen ist.

5. Instrument nach Anspruch 4, **dadurch gekennzeichnet, dass** die wenigstens eine Spule (34) des Hochfrequenzgenerators (20) mit einer Auskoppelspule (36, 36') in transformatorischer Kopplung angeordnet ist und dass die Auskoppelspule 36, 36') mit den beiden Elektroden (15, 16) verbunden ist.

6. Instrument einem der vorstehenden Ansprüche, **dadurch gekennzeichnet, dass** Hochfrequenzgenerator (20) mit dem Instrument (11) lösbar verbunden ist.

7. Instrument einem der vorstehenden Ansprüche, **dadurch gekennzeichnet, dass** der Hochfrequenzgenerator (20) ungeregelt ausgebildet ist und eine Strom/Spannungs-Charakteristik aufweist, die an die chirurgische Anwendung angepasst ist.

8. Instrument nach einem der vorstehenden Ansprüche, **dadurch gekennzeichnet, dass** der Versorgungsspannungseingang (22) mit mindestens einer Messeinrichtung (23) verbunden ist.

9. Instrument nach Anspruch 8, **dadurch gekennzeichnet, dass** die Messeinrichtung (23) eine Strommesseinrichtung (42) und/oder eine Spannungsmesseinrichtung (42) und/oder eine Leistungsmesseinrichtung (43) und/oder eine Frequenzmesseinrichtung (42, 43) umfasst.

10. Instrument nach einem der vorstehenden Ansprüche, **dadurch gekennzeichnet, dass** der Versorgungsspannungseingang (22) des Hochfrequenzgenerators (20) mit einer Spannungsmodulationseinrichtung (28) verbunden ist.

11. Instrument nach Anspruch 9 und 10, **dadurch gekennzeichnet, dass** die Spannungsmodulationseinrichtung (28) mit der Messeinrichtung (23) verbunden ist.

12. Einrichtung mit einem Instrument nach einem der vorstehenden Ansprüche,
mit einem Gerät (19), das eine Spannungsquelle (27) enthält, an die das Instrument (11) mittels eines Kabels (18) anschließbar ist.

13. Einrichtung mit einem Instrument (11) nach Anspruch 8, **dadurch gekennzeichnet**,
mit einem Gerät (19), das eine steuerbare Spannungsquelle (27) enthält, an die das Instrument (11) mittels eines Kabels (18) anschließbar ist,
wobei die Spannungsquelle (27) mit der Messeinrichtung verbindbar ist.

14. Einrichtung nach Anspruch 12, **dadurch gekennzeichnet, dass** in dem Gerät (19) eine Messeinrichtung (23) angeordnet ist, die mit der Spannungsquelle (27) verbunden ist.

15. Einrichtung nach Anspruch 14, **dadurch gekennzeichnet, dass** die Messeinrichtung (23) eine Strommesseinrichtung (42) und/oder eine Spannungsmesseinrichtung (42) und/oder eine Leistungsmesseinrichtung (43) und/oder eine Frequenzmesseinrichtung (42, 43) umfasst.

## Claims

1. An electrosurgical instrument (11) for the treatment of human or animal patients,
having at least two electrodes (15, 16) for applying current to biological tissue (17),
having at least one radio frequency generator (20) which is configured as a push-pull oscillator and comprises a supply voltage input (22) and a radio frequency output (21) connected to the electrode (15, 16), wherein the radio frequency generator (20) is configured to convert a temporally constant or temporally fluctuating supply voltage into a radio frequency AC voltage,
having a line (18) connectable to a voltage source (27) for supplying current to the radio frequency generator (20) at its supply voltage input (22),
**characterized in that** the push-pull oscillator comprises a push-pull flip-flop with two alternately switching transistors (T1, T2), to whose output electrodes a voltage amplifier is connected in each case.

2. The instrument according to claim 1, **characterized in that** the radio frequency generator (20) is configured to provide a voltage (UHF) having a frequency in the range between 100 kHz and 10 MHz.

3. The instrument according to any of the preceding claims, **characterized in that** the voltage amplifiers are transistors (T3, T4) in common gate or common base circuit.

4. The instrument according to any of the preceding claims, **characterized in that** the radio frequency generator (20) comprises a parallel resonant circuit (32) consisting of at least one coil (34) and at least one capacitor (33) that are connected in parallel to one another, and **in that** the radio frequency generator (20) comprises a decoupling circuit that is exclusively connected to the electrodes (15, 16) for electrically acting on the biological tissue (17).

5. The instrument according to claim 4, **characterized in that** the at least one coil (34) of the radio frequency generator (20) is arranged in transformer coupling with a decoupling coil (36, 36') and **in that** the decoupling coil (36, 36') is connected to the two electrodes (15, 16).

6. The instrument according to any of the preceding claims, **characterized in that** the radio frequency generator (20) is detachably connected to the instrument (11).

7. The instrument according to any of the preceding claims, **characterized in that** the radio frequency generator (20) is configured to be unregulated and comprises a current/voltage characteristic that is adapted to the surgical application.

8. The instrument according to any of the preceding claims, **characterized in that** the supply voltage input (22) is connected to at least one measurement device (23).

9. The instrument according to claim 8, **characterized in that** the measurement device (23) comprises a current measurement device (42) and/or a voltage measurement device (42) and/or a power measurement device (43) and/or a frequency measurement device (42, 43).

10. The instrument according to any of the preceding claims, **characterized in that** the supply voltage input (22) of the radio frequency generator (20) is connected to a voltage modulation device (28).

11. The instrument according to claim 9 and 10, **characterized in that** the voltage modulation device (28) is connected to the measurement device (23).

12. An arrangement comprising an instrument according to any of the preceding claims,
comprising an apparatus (19) having a voltage source (27) to which the instrument (11) can be connected by means of a cable (18).

13. An arrangement comprising an instrument (11) according to claim 8, **characterized by**
comprising an apparatus (19) that comprises a controllable voltage source (27) to which the instrument (11) can be connected by means of a cable (18),
wherein the voltage source (27) connectable to the measurement device.

14. The arrangement according to claim 12, **characterized in that** a measurement device (23) is arranged in the apparatus (19)m which is connected to the voltage source (27).

15. The arrangement according to claim 14, **characterized in that** the measurement device (23) comprises a current measurement device (42) and/or a voltage measurement device (42) and/or a power measurement device (43) and/or a frequency measurement device (42, 43).

## Revendications

1. Instrument électrochirurgical (11) destiné au traitement de patients humains et d'animaux,
comprenant au moins deux électrodes (15, 16) destinées à appliquer un courant à des tissus biologiques (17),
comprenant au moins un générateur haute fréquence (20) qui est réalisé sous forme d'oscillateur symétrique et présente une entrée de tension d'alimentation (22) et une sortie haute fréquence (21) raccordée à l'électrode (15, 16), le générateur haute fréquence (20) étant conçu pour transformer une tension d'alimentation constante dans le temps ou variant dans le temps en une tension alternative à haute fréquence,
comprenant un câble (18) pouvant être raccordé à une source de tension (27) et destiné à alimenter en courant le générateur haute fréquence (20), à son entrée de tension d'alimentation (22),
**caractérisé en ce que** l'oscillateur symétrique présente un étage de bascule symétrique comportant deux transistors (T1, T2) à commutation alternée, aux électrodes de sortie desquels est raccordé respectivement un amplificateur de tension.

2. Instrument selon la revendication 1, **caractérisé en ce que** le générateur haute fréquence (20) est conçu pour fournir une tension (UHF) d'une fréquence comprise entre 100 kHz et 10 MHz.

3. Instrument selon une des revendications précédentes, **caractérisé en ce que** les amplificateurs de tension sont des transistors (T3, T4) en montage à grille commune ou à base commune.

4. Instrument selon une des revendications précédentes, **caractérisé en ce que** le générateur haute fréquence (20) présente un circuit oscillant parallèle (32), constitué d'au moins une bobine (34) et d'au moins un condensateur (33) qui sont montés en parallèle l'un avec l'autre, et **en ce que** le générateur haute fréquence (20) présente un circuit de découplage qui est raccordé exclusivement aux électrodes (15, 16) en vue d'une action électrique sur les tissus biologiques (17).

5. Instrument selon la revendication 4, **caractérisé en ce que** la bobine (34), au nombre d'au moins une, du générateur haute fréquence (20) est installée dans un couplage de transformation avec une bobine de découplage (36, 36'), et **en ce que** la bobine de découplage (36, 36') est reliée aux deux électrodes (15, 16).

6. Instrument selon une des revendications précédentes, **caractérisé en ce que** le générateur haute fréquence (20) est relié de manière séparable à l'instrument (11).

7. Instrument selon une des revendications précédentes, **caractérisé en ce que** le générateur haute fréquence (20) est réalisé sans régulation et présente une caractéristique courant/tension qui est adaptée à l'application chirurgicale. ;

8. Instrument selon une des revendications précédentes, **caractérisé en ce que** l'entrée de tension d'alimentation (22) est reliée à au moins un dispositif de mesure (23).

9. Instrument selon la revendication 8, **caractérisé en ce que** le dispositif de mesure (23) comprend un dispositif de mesure de courant (42) et/ou un dispositif de mesure de tension (42) et/ou un dispositif de mesure de puissance (43) et/ou un dispositif de mesure de fréquence (42, 43).

10. Instrument selon une des revendications précédentes, **caractérisé en ce que** l'entrée de tension d'alimentation (22) du générateur haute fréquence (20) est reliée à un dispositif de modulation de tension (28).

11. Instrument selon la revendication 9 et 10, **caractérisé en ce que** le dispositif de modulation de tension (28) est relié au dispositif de mesure (23).

12. Equipement doté d'un instrument selon une des revendications précédentes,
comprenant un appareil (19) qui contient une source de tension (27) à laquelle l'instrument (11) peut être raccordé à l'aide d'un câble (18).

13. Equipement doté d'un instrument (11) selon la revendication 8, **caractérisé en ce qu'**il comprend
un appareil (19) qui contient une source de tension (27) pouvant être commandée, à laquelle l'instrument (11) peut être raccordé à l'aide d'un câble (18),
la source de tension (27) pouvant être raccordée au dispositif de mesure.

14. Equipement selon la revendication 12, **caractérisé en ce que** dans l'appareil (19), il est prévu un dispositif de mesure (23) qui est relié à la source de tension (27).

15. Equipement selon la revendication 14, **caractérisé en ce que** le dispositif de mesure (23) comprend un dispositif de mesure de courant (42) et/ou un dispositif de mesure de tension (42) et/ou un dispositif de mesure de puissance (43) et/ou un dispositif de mesure de fréquence (42, 43).
